# EUROPÄISCHE PATENTSCHRIFT

(11) **EP 3 228 271 B1**
(45) Veröffentlichungstag und Bekanntmachung des Hinweises auf die Patenterteilung: **25.11.2020**
(21) Anmeldenummer: 17165068.2
(22) Anmeldetag: 05.04.2017
(51) Int. Cl.: A61B 17/88, A61M 25/10

(54) **DOPPELBALLONKATHETER**
DOUBLE BALLOON CATHETER
CATHÉTER À DOUBLE BALLONNET

(30) Priorität: 08.04.2016 DE 202016101864 U
(43) Veröffentlichungstag der Anmeldung: 11.10.2017
(73) Patentinhaber: Joline GmbH & Co. KG, 72379 Hechingen (DE)
(72) Erfinder: SCHRECKLINGER, Wolfram, 72379 Hechingen (DE)
(74) Vertreter: Witte, Weller & Partner Patentanwälte mbB

(56) Entgegenhaltungen:
- EP-A1- 1 704 890
- US-A- 5 972 015
- US-A1- 2007 299 455
- US-A1- 2012 016 369
- US-A1- 2012 265 186
- US-A1- 2014 222 008
- US-A1- 2014 257 311

## Beschreibung

Die vorliegende Erfindung betrifft einen Ballonkatheter zum Komprimieren schwammartigen Knochenmaterials, wobei der Ballonkatheter einen Katheterkörper, und einen hierauf angeordneten ersten und zweiten Ballon aufweist, welche zum Komprimieren von schwammartigem Knochenmaterial inflatierbar sind.

Derartige Ballonkatheter, im medizinischen Gebiet auch Doppelballonkatheter genannt, sind im Stand der Technik allgemein bekannt.

So werden bspw. Doppelballonkatheter zur Behandlung von verengten (Blut-)Gefäßen, wie bspw. in der Angioplastie eingesetzt, oder um endoluminale Prothesen zu entfalten.

In der Angioplastie werden Ballonkatheter, in der Regel fast immer von der Leiste aus, über einen Führungsdraht und Führungskatheter in die Stenose (Engstelle) platziert und mit Druck (8-12 bar) über ein geeignetes Fluid aufgeblasen. Das Aufblasen bzw. Aufpumpen weitet die Stenose radial nach außen und drückt sie gegen die Gefäßwand zusammen, so dass dessen Querschnittsfläche erweitert wird. Damit wird ein akzeptabler Blutdurchfluss erreicht, die Engstelle beseitigt und eine Operation vermieden.

Bei einem Einsatz von Ballon-dilatierbaren Stents werden diese ebenfalls über einen Einführkatheter im komprimierten Zustand in das Gefäß eingeführt, und durch einen innerhalb des Stents liegenden Ballon bzw. über die Zuführung von Fluid in den Ballon und dessen radialer Expansion ebenfalls aufgedehnt.

Ballonkatheter werden außerdem im Gebiet der Kyphoplastie, also der Behandlung von Wirbelbrüchen, eingesetzt. Wirbelbrüche sind insbesondere osteoporotisch, traumatisch oder tumorös bedingt, und werden gegenwärtig durch die Verfahren der Vertrebroplastie oder Kyphoplastie behandelt.

Während bei der Vertebroplastie eine Hohlnadel durch die Bogenwurzeln des betroffenen Wirbels eingebracht und anschließend Knochenzement in den zu behandelnden Wirbelkörper eingespritzt wird, der in kurzer Zeit aushärtet, wird bei dem ebenfalls minimalinvasiven Verfahren der Kyphoplastie zunächst mittels einer Kanüle ein kollabierter Ballon in den zu behandelnden Wirbelkörper eingeführt. Durch Inflatieren/Expandieren des Ballons kann der zusammengebrochene Wirbel teilweise wieder aufgerichtet und anschließend fixiert werden, indem in die entstandene Kavität(en) Knochenzement eingespritzt oder anderweitig eingeführt wird, der innerhalb weniger Minuten aushärtet und damit den gebrochenen Wirbel stabilisiert.

Je nach Technik werden kleinere oder größere Zugänge zum Wirbelkörper benötigt. Neuere Techniken benötigen lediglich kleinere Zugänge und legen nur sehr kleine Kavitäten bzw. Gänge an. Dadurch kann der eingeführte Knochenzement gleichmäßig verteilt und die gesunde Spongiosa mit dem Knochenzement verzahnt werden.

Die hierfür notwendigen Instrumente umfassen dabei regelmäßig zumindest eine Zugangs- bzw. Arbeitskanüle, mit der der perkutane Zugang zum Wirbelkörper bereitgestellt wird, eine Baugruppe aus Knochenzement-Kanüle und Stempel, mit welchem der in der Knochenzement-Kanüle vorliegende Knochenzement aus dieser in den Wirbelkörper vorgeschoben werden kann, sowie ggf. ein Instrument zum Ausbilden einer Kavität im Wirbelkörper, bspw. einem Ballonkatheter. Die Einführung der Instrumente in den Wirbelkörper erfolgt dabei regelmäßig unter fluoroskopischer/röntgonologischer Kontrolle.

So ist bspw. aus der WO 00/09024 A1 ein solches System bekannt, das eine solche Zugangskanüle, ein eine Kavität ausbildendes Instrument und eine Baugruppe aus Kanüle und Stopfer umfasst.

Da sich die Anforderungen an die Schaffung von Hohlräumen im Wirbelkörper von Patient zu Patient unterscheiden, bspw. Aufgrund der unterschiedlichen Defekte im Wirbelkörper, oder unterschiedlich dichtem Knochenmaterial, sowie unterschiedlicher Dimensionen, sowie aufgrund der chirurgischen Besonderheit bei einem Eingriff am Wirbelkörper, sind an die hier eingesetzten Katheter besondere Anforderungen gestellt, die von den im Stand der Technik bekannten Kathetern nicht immer erfüllt werden.

Aufgabe der vorliegenden Erfindung ist es daher, einen Ballonkatheter bereitzustellen, der gezielt auf den Patienten bzw. die Gegebenheiten des zu behandelnden Wirbelkörpers abgestimmt ist, eine schonende Behandlung und einfache Handhabung ermöglicht.

Erfindungsgemäß wird diese Aufgabe gelöst durch einen Ballonkatheter zum Komprimieren schwammartigen Knochenmaterials, wobei der Ballonkatheter folgendes aufweist: einen länglichen flexiblen Katheterkörper mit einem proximalen und einem distalen Ende, sowie einer sich zwischen dem proximalen und distalen Ende erstreckenden Längsachse, und einem distalen und proximalen Endbereich; ein Anschlusselement, das mit dem proximalen Endbereich des Katheterkörpers fest verbunden ist, und das zumindest zwei oder weitere Anschlüsse aufweist; ein erstes in- und deflatierbares Ballonelement, das auf einen ersten Durchmesser zu einem inflatierten Zustand inflatierbar ist, und das koaxial um den Katheterkörper an dessen distalem Ende angeordnet ist, und wobei das erste Ballonelement über ein erstes Lumen über ein darin geführtes Fluid inflatierbar ist, wobei das erste Lumen sich vom distalen Endbereich entlang der Längsachse des Katheterkörpers und zum proximalen Ende in einen ersten Anschluss erstreckt, wobei das erste Lumen eine sich in das Innere des ersten Ballonelements öffnende Öffnung aufweist; und wobei ferner das erste Ballonelement einen ersten distalen Ballonbereich einen ersten proximalen Ballonbereich und einen dazwischen liegenden ersten mittleren Ballonbereich aufweist, wobei das erste Ballonelement über den ersten distalen und ersten proximalen Ballonbereich am Katheterkörper fixiert ist, und wobei das erste Ballonelement im ersten mittleren Ballonbereich eine erste Einengung aufweist, die den Ballonelement-Umfang im inflatierten Zustand im Vergleich zu den unmittelbar rechts und links von der Einengung liegenden Bereichen reduziert; ein zweites in- und deflatierbares Ballonelement, das auf einen zweiten Durchmesser in einen inflatierten Zustand inflatierbar ist, und das koaxial um den Katheterkörper unmittelbar benachbart und proximal zum ersten Ballonelement angeordnet ist, und wobei das zweite Ballonelement über ein zweites Lumen über ein darin geführtes Fluid in- und deflatierbar ist, wobei das zweite Lumen sich vom distalen Endbereich entlang der Längsachse des Katheterkörpers und zum proximalen Ende in einen zweiten Anschluss erstreckt, wobei das zweite Lumen eine sich in das Innere des zweiten Ballonelements öffnende Öffnung aufweist; und wobei ferner das zweite Ballonelement einen zweiten distalen Ballonbereich, einen zweiten proximalen Ballonbereich und einen dazwischen liegenden zweiten mittleren Ballonbereich aufweist, wobei das zweite Ballonelement über den zweiten distalen und zweiten proximalen Ballonbereich am Katheterkörper fixiert ist, und wobei das zweite Ballonelement im zweiten mittleren Ballonbereich eine erste Einengung aufweist, die den Ballonelement-Umfang im inflatierten Zustand im Vergleich zu den unmittelbar rechts und links von der Einengung liegenden Bereichen reduziert; dabei grenzen der erste proximale Ballonbereich des ersten Ballonelements und der zweite distale Ballonbereich des zweiten Ballonelements unmittelbar und bündig aneinander an, oder überlappen oder sind einstückig ausgebildet.

Die Erfindung betrifft außerdem ein Set zur Behandlung von Wirbelkörpererkrankungen oder -verletzungen, das den erfindungsgemäßen Ballonkatheter aufweist.

Die der Erfindung zugrunde liegende Aufgabe wird auf diese Weise vollkommen gelöst. Insbesondere ist es mit dem Ballonkatheter möglich, die beiden Ballonelemente unabhängig voneinander zu inflatieren, wodurch die Behandlung spezifisch auf den Wirbelkörper abgestimmt werden kann. Der Ballonkatheter ist außerdem einfach zu handhaben, wodurch wiederum die Behandlungsdauer verkürzt und der Patient insgesamt schonender behandelt werden kann.

In der Praxis wird zunächst ein transdermaler Zugang zu dem zu behandelnden Wirbelkörper gelegt, und eine Einführ- bzw. Arbeitskanüle gelegt, über welche der Wirbelkörper für verschiedene Geräte zugänglich ist. Der erfindungsgemäße Ballonkatheter wird durch diese Einführkanüle in Richtung des Wirbelkörpers vorgeschoben, und im Wirbelkörper nach Bedarf zur Schaffung von Hohlräumen inflatiert.

Gemäß einer bevorzugten Ausführungsform ist dabei vorgesehen, wenn der Katheterkörper Markierungen in einem Bereich zwischen dem proximalen und distalen Endbereich aufweist, zum Anzeigen der Position des Ballonkatheters in einer Einführkanüle.

Diese Ausführungsform hat den Vorteil, dass der behandelnde Chirurg damit das Vorschieben des Ballonkatheters in den Wirbelkörper unmittelbar nachvollziehen, und die Weite des Vorschiebens entsprechend einfach regulieren kann.

Dabei ist bevorzugt, wenn drei, hintereinander und in einem Abstand voneinander angeordnete Markierungen im Bereich zwischen dem proximalen und dem distalen Endbereich vorgesehen, sind, nämlich eine distale, eine mittlere und eine proximale Markierung, wobei die distale Markierung zum Anzeigen des Austritts des Ballonkatheters aus der Einführkanüle ausgebildet ist, die mittlere zum Anzeigen der Freisetzung des distalen Ballonelements, und die proximale Markierung zum Anzeigen der Freisetzung/Inflation beider Ballonelemente ausgebildet ist. Die Markierungen sind dabei bevorzugt röntgendichte Ringe am Katheterkörper, die entsprechend den Distanzen und in Abstimmung auf die Einführkanüle am Katheterkörper angebracht sind.

In einer Weiterbildung des erfindungsgemäßen Ballonkatheters ist im proximalen Endbereich im Übergang vom Katheterkörper zum Anschlusselement ein über dem Katheterkörper koaxial angeordnetes, den Katheterkörper im proximalen Endbereich versteifendes Knickschutz-Element angeordnet ist, das mit dem Anschlusselement fest verbunden ist.

Diese Ausführungsform hat den Vorteil, dass damit ein Abknicken des Anschlusselements vermieden und damit bspw. die ungehinderte Zuleitung von Fluid und insgesamt eine störungsfreie Handhabung des Ballonkatheters gesichert ist.

In einer weiteren Ausführungsform umfasst zumindest der erste und der zweite Anschluss Vakuumventile.

Gemäß einer weiteren Ausbildung des erfindungsgemäßen Ballonkatheters umfasst dieser ferner ein Stylet-Element, welches über das erste oder zweite Lumen zum Versteifen des Katheterkörpers in diesem fixiert ist.

Diese Ausführungsform hat den Vorteil, dass damit ein Knicken des Katheterkörpers, insbesondere beim Einführen über eine Einführkanüle, sowie beim Inflatieren der Ballonelemente vermieden wird, und die Bearbeitung insgesamt vereinfacht und beschleunigt wird.

In einer bevorzugten Ausführungsform ist bei dem erfindungsgemäßen Ballonkatheter vorgesehen, wenn das erste Ballonelement in seinem distalen Ballonbereich durch eine teilweise Umstülpung des distalen Ballonbereichs nach Innen und eine Fixierung des nach innen gestülpten distalen Ballonbereichs am Katheterkörper fixiert ist.

Bei dieser Ausführungsform wird - in anderen Worten - der distale Ballonbereich nach Innen, d.h. zum Katheterkörper hin umgeschlagen, und dadurch praktisch mit der Außenseite des Ballons am Katheterkörper fixiert. Da der distale und der proximale Ballonbereiche vorzugsweise am Katheterkörper angeschweißt werden, wird der gesamte distale Ballonbereich fixiert.

Diese Ausführungsform hat den Vorteil, dass dadurch eine atraumatische Spitze geschaffen wird.

In einer weiteren Ausführungsform ist bei dem erfindungsgemäßen Ballonkatheter bevorzugt, wenn der proximale Ballonbereich des ersten Ballonelements und der distale Ballonbereich des zweiten Ballonelements überlappend an dem Katheterkörper fixiert, vorzugsweise angeschweißt, sind.

Erfindungsgemäße bzw. gemäß einer vorteilhaften Ausführungsform weist dabei das erste und/oder zweite Ballonelement ein Material auf, oder besteht aus diesem, das ausgewählt ist aus Polyurethan, Polyethylen, Teflon, Nylon.

Diese Materialien sind im Stand der Technik als medizinisch verträglich bekannt, und eignen sich als inerte Materialien zum Gebrauch im erfindungsgemäßen Ballonkatheter.

Insgesamt ist bei einer bevorzugten Ausführungsform vorgesehen, wenn das erste Lumen einen im Wesentlichen kreisförmigen Querschnitt aufweist, und weiter, wenn das zweite Lumen einen im Wesentlichen langlochartigen Querschnitt aufweist.

Dabei bedeutet "im Wesentlichen", dass die Form der Querschnitte nicht exakt kreis- bzw. langlochförmig sein muss, sondern auch hiervon abweichende unregelmäßigere Formen, die aber allgemein noch als kreisförmig bzw. langlochförmig verstanden werden. Dabei bedeutet "langlochartig" jede Form einer Öffnung, die eher rechteckig als quadratisch ist, wenn auch mit vorzugsweise abgerundeten Ecken.

In einer bevorzugten Ausführungsform weist der proximale Ballonbereich des ersten Ballonelements und/oder der proximale Ballonbereich des zweiten Ballonelements einen konusförmigen Verlauf auf, wobei das reduzierte Ende des konusförmigen Verlaufs in die proximale Richtung weisend angeordnet ist.

Diese Ausführungsform hat den Vorteil, dass damit eine Materialsparende Bauweise erreicht wird, was sowohl hinsichtlich der Kosten, als auch in der Handhabung als solches vorteilhaft ist, da kein überflüssiges Material die Handhabung des Ballonkatheters stören kann.

Bei dem erfindungsgemäße Ballonkatheter ist ferner bevorzugt, wenn er derart dimensioniert ist, dass er über eine Einführkanüle und durch diese hindurch mit seinem die Ballonelemente umfassenden distalen Endbereich in einen Wirbelkörper vorschiebbar und in dem Wirbelkörper zum Komprimieren von schwammartigem Knochenmaterial ausgebildet ist.

Dem Fachmann wird ausgehend von der hierin beschriebenen Lehre klar sein, dass die Einführkanüle und der Ballonkatheter insbesondere im Hinblick auf ihre jeweiligen Durchmesser und Längen aufeinander abgestimmt sein müssen.

Die vorliegende Erfindung betrifft ferner die Verwendung des erfindungsgemäßen Ballonkatheters zur Verwendung in der Behandlung von Wirbelkörpererkrankungen oder -verletzungen, sowie ein Set zur Behandlung von Wirbelkörpererkrankungen oder -verletzungen, das den erfindungsgemäßen Ballonkatheter sowie zumindest eines der folgenden umfasst: einen Ballonadapter, eine Einführkanüle, eine Volumenspritze, eine Vorrichtung zur Ausbildung eines Zugangs zum Wirbelkörper.

Ganz allgemein wird im chirurgischen bzw. medizinischen Gebiet, in dem Instrumente und Geräte zur Behandlung eines Patienten eingesetzt werden - wie auch vorliegend -, das Ende eines Instruments oder Geräts, das der behandelnde Chirurg handhabt, bzw. das näher an ihm ist, als "Proximal" bezeichnet, während das Ende des Geräts, das weiter vom Chirurg entfernt ist, als "Distal" bezeichnet.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:

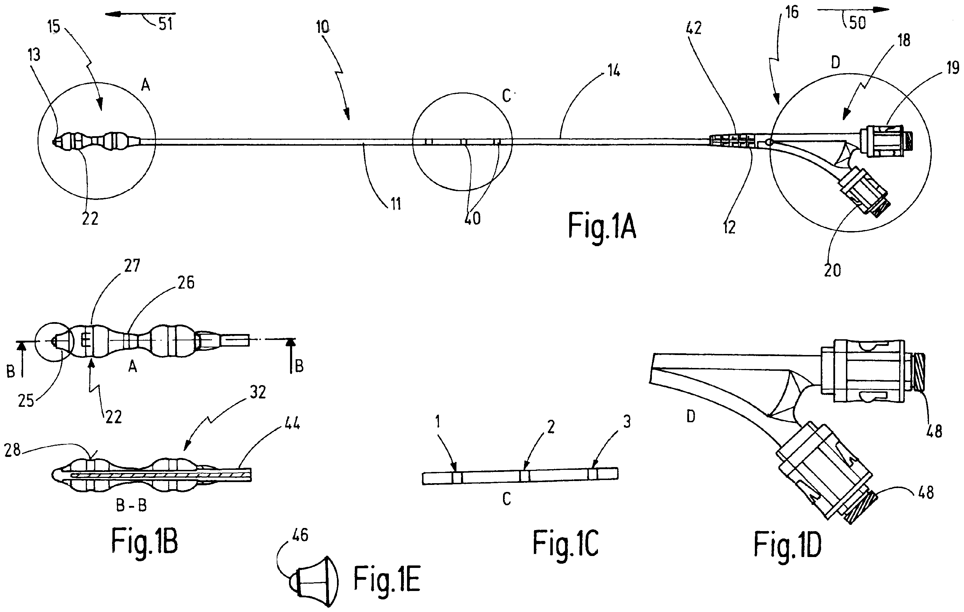

- **Fig. 1**: eine schematische Darstellung einer Ausführungsform eines erfindungsgemäßen Ballonkatheters in einer Übersichtsdarstellung **(A),** sowie Detailansichten der Ballonelemente, in seitlicher Draufsicht und entlang der Linie B-B im Ballonelement umfassenden Abschnitt **(B);** eine Detailansicht des Markierungen-tragenden Abschnitts **(C);** eine Detailansicht des Anschlussbereichs **(D),** sowie eine Detailansicht der atraumatischen Spitze **(E);**

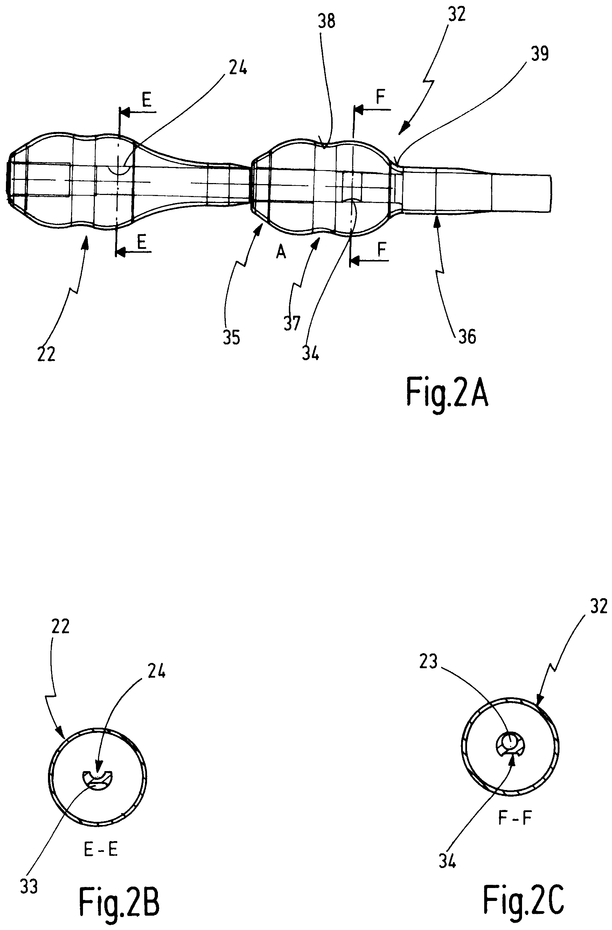

- **Fig. 2**: eine stark vergrößerte Darstellung des die Ballonelemente tragenden Abschnitts des Ballonkatheters **(A),** sowie von Querschnittsdarstellungen entlang des Schnitts E-E **(B)** bzw. F-F **(C).**

In den Figuren sind gleiche Merkmale mit den gleichen Bezugszeichen versehen, wobei aus Gründen der Übersichtlichkeit nicht in allen Figuren alle Bezugszeichen angegeben sind.

In Fig. 1A ist mit 10 insgesamt ein Ballonkatheter bezeichnet, der einen flexiblen, länglichen Katheterkörper 11 mit einem proximalen Ende 12 und einem distalen Ende 13 aufweist, sowie eine sich zwischen dem proximalen Ende 12 und dem distalen Ende 13 erstreckenden Längsachse 14. Ferner umfasst der Ballonkatheter einen proximalen Endbereich 16 und einen distalen Endbereich 15.

Ein Anschlusselement 18 ist mit dem proximalen Endbereich 16 verbunden, mit einem ersten Anschlusselement 20 und einem zweiten Anschlusselement 19.

Wie Fig. 1A ferner zu entnehmen ist, ist am distalen Ende 13 des Ballonkatheters 10 ein erstes Ballonelement 22 angebracht, das an diesem Ende 13 koaxial um den Katheterkörper 11 angeordnet ist, und das über Zuleitung eines Fluids über ein erstes Lumen 23 inflatierbar ist (siehe Fig. 2B und 2C). Das erste Lumen 23 weist eine sich in das Innere des ersten Ballonelements 22 öffnende Öffnung 24 auf (siehe Fig. 2A; 2B), über welche das Fluid in das Innere des Ballonelements 22 fließt und diesen dadurch inflatiert. Das erste Lumen 23 erstreckt sich vom distalen Endbereich 15 entlang der Längsachse 14 des Katheterkörpers 11 und zum proximalen Ende 12 in das erste Anschlusselement bzw. in den ersten Anschluss 20.

Aus der in Fig. 1B gezeigten Detaildarstellung des distalen Endbereichs 15 geht die Bauweise des Ballonelements 22 genauer hervor. Fig. 1B zeigt in der oberen Darstellung eine seitliche Draufsicht auf den Endbereich 15, und in der unteren Darstellung einen Schnitt entlang der Achse B-B. Das Ballonelement 22 umfasst einen ersten distalen Ballonbereich 25, einen ersten proximalen Ballonbereich 26 und einen dazwischen liegenden ersten mittleren Ballonbereich 27. Die Ballonbereiche 25, 27, 26 sind in dieser Reihenfolge von distal nach proximal angeordnet, wobei, wie weiter oben ausgeführt, mit "distal" diejenige Richtung bzw. dasjenige Ende eines länglichen Instruments bezeichnet wird, die/das vom Anwender weiter weg liegt als das andere Ende/ die andere Richtung. In Fig. 1 ist mit dem Pfeil 50 die proximale Richtung angegeben, und mit dem Pfeil 51 die distale Richtung angezeigt.

Das Ballonelement 22 ist über den ersten distalen und ersten proximalen Ballonbereich 25, 26, am Katheterkörper fixiert, vorzugswiese aufgeschrumpft, angeschweißt, angeklebt oder anderweitig fest mit diesem verbunden. Fig. 1B, insbesondere in der unteren Darstellung, ist ferner zu entnehmen, dass der erste mittlere Ballonbereich 27 eine erste Einengung 28 aufweist, die den Ballonelement-Umfang im inflatierten Zustand im Vergleich zu den unmittelbar rechts und links von der Einengung 28 liegenden Bereichen des mittleren Ballonbereichs 27 reduziert.

Fig. 1B ist ferner, in der unteren Darstellung, das Vorliegen eines Stylet-Element 44 gezeigt, das über das Lumen 23 des Katheterkörpers 11 zu dessen Versteifung eingeführt werden kann. Alternativ kann das Stylet auch über das Lumen 33 eingeführt werden. Das Lumen 23 weist über seine Länge einen im Wesentlichen kreisförmigen Querschnitt auf.

Die Figuren 1 und 2 zeigen ferner, das ein zweites in- und deflatierbares Ballonelement 32 vorgesehen ist, das - unabhängig vom ersten Ballonelement 22 - auf einen zweiten Durchmesser inflatiert werden kann. Auch das zweite Ballonelement 32 ist koaxial um den Katheterköprer 11 angeordnet, und ist über ein zweites Lumen 33 über ein darin geführtes Fluid inflatierbar. Es versteht sich, dass beide Ballonelemente 22 und 32, durch Ableiten bzw. Absaugen des Fluids jeweils - auch unabhängig voneinanderdeflatierbar sind, also entleert werden können.

Das zweite Lumen 33 erstreckt sich dabei von distalen Endbereich 15 entlang der Längsachse 14 des Katheterkörpers 11 bis zum proximalen Ende 12 in einen zweiten Anschluss bzw. in das zweite Anschlusselement 19. Das zweite Lumen 33 weist eine sich in das Innerer des zweiten Ballonelements 32 öffnende Öffnung 34 auf (siehe Fig. 2A, 2C). Über seine Gesamtlänge gesehen weist das zweite Lumen 32 einen im Wesentlichen langlochförmigen Querschnitt auf.

Wie insbesondere Fig. 2 zu entnehmen ist, weist das zweite Ballonelement 32 ferner einen zweiten distalen Ballonbereich 35, eine zweiten proximalen Ballonbereich 36 und einen dazwischen liegenden zweiten mittleren Ballonbereich 37 auf. Das Ballonelement 32 ist über den zweiten distalen und zweiten proximalen Ballonbereich 35 und 36 am Katheterkörper 11 fixiert. Ferner weist der mittlere Ballonbereich 37 eine erste Einengung 38 auf, die den Ballonelement-Umfang im inflatierten Zustand im Verglich zu den unmittelbar rechts und links von der Einengung 38 liegenden Bereichen reduziert.

In Fig. 2A ist ferner gezeigt, dass das zweite Ballonelement 32 im zweiten proximalen Ballonbereich 36 eine zweite Einengung 39 aufweist, über welche der Umfang des zweiten Ballonelements 32 proximal der zweiten Einengung 39 reduzierter ist als Umfang des zweiten Ballonelements 32 distal der zweiten Einengung 39.

Den Fig. 1 und 2 ist ferner zu entnehmen, dass der erste proximale Ballonbereich 26 des ersten Ballonelements 22 und der zweite distale Ballonbereich 35 des zweiten Ballonelements 32 unmittelbar und bündig aneinander angrenzen. Alternativ können die beiden Ballonelemente 22 und 32 auch überlappen oder einstückig ausgebildet sein.

In Fig. 1 ist ferner gezeigt, dass der Katheterkörper Markierungen 40 im Bereich zwischen dem proximalen und dem distalen Endbereichen 15, 16 aufweist, die auch proximal zu den Ballonelementen 22, 32 am Katheterkörper 11 angeordnet sind.

Diese Markierungen 40 stellen Einführmarkierungen dar, die die Position des Katheters 10 in einer Einführkanüle (nicht gezeigt) anzeigen sollen. In Fig. 1A zeigen die drei Markierungen, bei einer Betrachtung von links nach rechts, 1.) die Position an, bei der die Katheterspitze 46 aus der Kanüle austritt, 2.) die Position, in der das erste Ballonelement 22 aus der Kanüle freigesetzt ist, und 3.) die Position, in der beide Ballonelemente 22, 32 aus der Kanüle herausgeschoben sind.

Fig. 1A zeigt ferner, dass im proximalen Endberiech 16, im Übergang vom Katheterkörper 11 zum Anschlusselement 18, ein über dem Katheterkörper 11 koaxial angeordnetes, den Katheterkörper 11 im proximalen Endbereich 16 versteifendes Knickschutz-Element 42 angeordnet ist. Dieses Knickschutz-Element 42 ist mit dem Anschlusselement 18 fest verbunden, und schützt davor, dass bei einer Handhabung des Katheters 10 das Anschlusselement 18 vom Katheterkörper 11 abknickt, wodurch es zum Unterbrechungen der Fluidzufuhr und zu Schwierigkeiten beim Einführen in eine Arbeitskanüle kommen kann.

In Fig. 1E ist in einer Detailansicht das distale Ende 13 des Katheters 10 gezeigt, das die atraumatische Spitze 46 umfasst.

Fig. 1D zeigt eine Detailansicht des Anschlusselements 18, wobei hier noch Vakuumventile 48 bezeichnet sind, die jeweils am ersten bzw. zweiten Anschluss 20, 19 angeordnet sind, und zum Deflatieren der Ballonelemente 22, 32 vorgesehen sind.

Die Figuren 2B und 2C zeigen jeweils Querschnitte entlang der Linien E-E (2B) und F-F (2C), und verdeutlichen die Lage der Öffnungen 24, 34 der Lumen 22, 32.

Fig. 2A ist ferner zu entnehmen, dass der distale Ballonbereich 25 des ersten, distalen Ballonelements nach Innen, d.h. zum Katheterkörper 11 hin, umgestülpt bzw. umgeschlagen und am Katheterkörper 11 fixiert ist. Dadurch wird das Ballonelement 22 über seinen distalen Ballonbereich 25 praktisch im Inneren des Ballonelements 22 am Katheterkörper 11 fixiert.

Der proximale Ballonbereich 26 des ersten, distalen Ballonelements 22 und der distale Ballonbereich 35 des zweiten Ballonelements 32 sind überlappend am Katheterkörper fixiert.

## Patentansprüche

1. Ballonkatheter (10) zum Komprimieren schwammartigen Knochenmaterials, wobei der Ballonkatheter (10) folgendes aufweist:
einen länglichen flexiblen Katheterkörper (11) mit einem proximalen (12) und einem distalen (13) Ende, sowie einer sich zwischen dem proximalen (12) und distalen (13) Ende erstreckenden Längsachse (14), und einem distalen (15) und proximalen (16) Endbereich;
einem Anschlusselement (18), das mit dem proximalen Endbereich (16) des Katheterkörpers (11) fest verbunden ist, und das zumindest zwei oder weitere Anschlüsse (19, 20) aufweist;
- ein erstes in- und deflatierbares Ballonelement (22), das auf einen ersten Durchmesser zu einem inflatierten Zustand inflatierbar ist, und das koaxial um den Katheterkörper (11) an dessen distalem Ende (13) angeordnet ist, und wobei das erste Ballonelement (22) über ein erstes Lumen (23) über ein darin geführtes Fluid inflatierbar ist, wobei das erste Lumen (23) sich vom distalen Endbereich (15) entlang der Längsachse (14) des Katheterkörpers (11) und zum proximalen Ende (12) in einen ersten Anschluss (20) erstreckt, wobei das erste Lumen (23) eine sich in das Innere des ersten Ballonelements (22) öffnende Öffnung (24) aufweist; und wobei ferner das erste Ballonelement (22) einen ersten distalen Ballonbereich (25), einen ersten proximalen Ballonbereich (26) und einen dazwischen liegenden ersten mittleren Ballonbereich (27) aufweist, wobei das erste Ballonelement (22) über den ersten distalen (25) und ersten proximalen (26) Ballonbereich am Katheterkörper (11) fixiert ist, und wobei das erste Ballonelement (22) im ersten mittleren Ballonbereich (27) eine erste Einengung (28) aufweist, die den Ballonelement-Umfang im inflatierten Zustand im Vergleich zu den unmittelbar rechts und links von der Einengung (28) liegenden Bereichen des mittleren Ballonbereichs (27) reduziert;
- ein zweites in- und deflatierbares Ballonelement (32), das auf einen zweiten Durchmesser in einen inflatierten Zustand inflatierbar ist, und das koaxial um den Katheterkörper (11) unmittelbar benachbart und proximal zum ersten Ballonelement (22) angeordnet ist, und wobei das zweite Ballonelement (32) über ein zweites Lumen (33) über ein darin geführtes Fluid inflatierbar ist, wobei das zweite Lumen (33) sich vom distalen Endbereich (15) entlang der Längsachse (14) des Katheterkörpers (11) und zum proximalen Ende (12) in einen zweiten Anschluss (19) erstreckt, wobei das zweite Lumen (33) eine sich in das Innere des zweiten Ballonelements (32) öffnende Öffnung (34) aufweist; und wobei ferner das zweite Ballonelement (32) einen zweiten distalen Ballonbereich (35), einen zweiten proximalen Ballonbereich (36) und einen dazwischen liegenden zweiten mittleren Ballonbereich (37) aufweist, wobei das zweite Ballonelement (32) über den zweiten distalen (35) und zweiten proximalen (36) Ballonbereich am Katheterkörper (11) fixiert ist, und wobei das zweite Ballonelement (32) im zweiten mittleren Ballonbereich (37) eine erste Einengung (38) aufweist, die den Ballonelement-Umfang im inflatierten Zustand im Vergleich zu den unmittelbar rechts und links von der Einengung (38) liegenden Bereichen reduziert
wobei der erste proximale Ballonbereich (26) des ersten Ballonelements (22) und der zweite distale Ballonbereich (35) des zweiten Ballonelements (32) unmittelbar und bündig aneinander angrenzen, oder überlappen oder einstückig ausgebildet sind.

2. Ballonkatheter (10) nach Anspruch 1, **dadurch gekennzeichnet, dass** der Katheterkörper (11) Markierungen (40) in einem Bereich zwischen dem proximalen (16) und distalen (15) Endbereich aufweist, zum Anzeigen der Position des Ballonkatheters (10) in einer Einführkanüle.

3. Ballonkatheter (10) nach Anspruch 1 oder 2, **dadurch gekennzeichnet, dass** im proximalen Endbereich (16) im Übergang vom Katheterkörper (11) zum Anschlusselement (18) ein über dem Katheterkörper (11) koaxial angeordnetes, den Katheterkörper (11) im proximalen Endbereich (16) versteifendes Knickschutz-Element (42) angeordnet ist, das mit dem Anschlusselement (18) fest verbunden ist.

4. Ballonkatheter (10) nach einem der vorstehenden Ansprüche, **dadurch gekennzeichnet, dass** zumindest der erste (20) und der zweite (19) Anschluss Vakuumventile umfassen.

5. Ballonkatheter (10) nach einem der vorstehenden Ansprüche, **dadurch gekennzeichnet, dass** er ferner ein Stylet-Element (44) umfasst, welches über das erste (23) oder zweite (33) Lumen zum Versteifen des Katheterkörpers (11) in diesem fixiertist.

6. Ballonkatheter (10) nach einem der vorstehenden Ansprüche, **dadurch gekennzeichnet, dass** das erste Ballonelement (22) in seinem distalen Ballonbereich (25) durch eine teilweise Umstülpung des distalen Ballonbereichs (25) nach Innen und eine Fixierung des nach innen gestülpten distalen Ballonbereichs am Katheterkörper (11) fixiert, vorzugsweise angeschweißt, ist.

7. Ballonkatheter (10) nach einem der vorstehenden Ansprüche, **dadurch gekennzeichnet, dass** der proximale Ballonbereich (26) des ersten Ballonelements (22) und der distale Ballonbereich (35) des zweiten Ballonelements (32) überlappend an dem Katheterkörper (11) fixiert, vorzugsweise angeschweißt, sind.

8. Ballonkatheter (10) nach einem der vorstehenden Ansprüche, **dadurch gekennzeichnet, dass** das erste und/oder zweite Ballonelement (22; 32) ein Material aufweist oder aus diesem besteht, das ausgewählt ist aus Polyurethan, Polyethylen, Teflon, Nylon.

9. Ballonkatheter (10) nach einem der vorstehenden Ansprüche, **dadurch gekennzeichnet, dass** das erste Lumen (23) einen im Wesentlichen kreisförmigen Querschnitt aufweist.

10. Ballonkatheter (10) nach einem der vorstehenden Ansprüche, **dadurch gekennzeichnet, dass** das zweite Lumen (33) einen im Wesentlichen langlochartigen Querschnitt aufweist.

11. Ballonkatheter (10) nach einem der vorstehenden Ansprüche, **dadurch gekennzeichnet, dass** der proximale Ballonbereich (26) des ersten Ballonelements (22) und/oder der proximale Ballonbereich (36) des zweiten Ballonelements (32) einen konusförmigen Verlauf aufweist, wobei das reduzierte Ende des konusförmigen Verlaufs in die proximale Richtung (50) weisend angeordnet ist.

12. Ballonkatheter (10) nach einem der vorstehenden Ansprüche, **dadurch gekennzeichnet, dass** er derart dimensioniert ist, dass er über eine Einführkanüle und durch diese hindurch mit seinem die Ballonelemente (22; 32) umfassenden distalen Endbereich (15) in einen Wirbelkörper vorschiebbar und in dem Wirbelkörper zum Komprimieren von schwammartigem Knochenmaterial ausgebildet ist.

13. Set zur Behandlung von Wirbelkörpererkrankungen oder -verletzungen, **dadurch gekennzeichnet, dass** es den Ballonkatheter (10) nach einem der vorstehenden Ansprüche sowie zumindest eines der folgenden umfasst: einen Ballonadapter, eine Einführkanüle, eine Volumenspritze, eine Zugangsvorrichtung.

## Claims

1. Balloon catheter (10) for compressing spongy bone material, said balloon catheter (10) comprising
- an elongated flexible catheter body (11) having a proximal end (12) and a distal end (13), a longitudinal axis (14) extending between the proximal end (12) and the distal end (13), and a distal end portion (15) and a proximal (16) end portion;
- a connecting element (18) which is firmly connected to the proximal end portion (16) of the catheter body (11) and which has at least two or more further connecting ports (19, 20);
- a first inflatable and deflatable balloon member (22) inflatable to a first diameter to an inflated state, and which is arranged coaxially around the catheter body (11) at its distal end (13), and wherein the first balloon member (22) is inflatable via a first lumen (23) via a fluid conducted therein, the first lumen (23) extending from the distal end portion (15) along the longitudinal axis (14) of the catheter body (11) and to the proximal end (12) into a first connecting port (20), the first lumen (23) having an opening (24) opening into the interior of the first balloon member (22); and further wherein the first balloon member (22) has a first distal balloon portion (25), a first proximal balloon portion (26) and a first central balloon portion (27) therebetween, the first balloon member (22) being fixed to the catheter body (11) via the first distal balloon portion (25) and the first proximal balloon portion (26), and wherein the first balloon element (22) has a first constriction (28) in the first central balloon portion (27) which reduces the balloon element circumference in the inflated state in comparison with the portions of the central balloon portion (27) lying immediately to the right and left of the constriction (28);
- a second inflatable and deflatable balloon member (32) inflatable to a second diameter to an inflated state, said second balloon member (32) being arranged coaxially around the catheter body (11) immediately adjacent and proximal to the first balloon member (22), and said second balloon member (32) being inflatable via a second lumen (33) via a fluid carried therein, the second lumen (33) extending from the distal end portion (15) along the longitudinal axis (14) of the catheter body (11) and to the proximal end (12) into a second connecting port (19), the second lumen (33) having an opening (34) opening into the interior of the second balloon member (32); and further wherein the second balloon member (32) has a second distal balloon portion (35), a second proximal balloon portion (36) and a second central balloon portion (37) therebetween, the second balloon member (32) being fixed to the catheter body (11) via the second distal balloon portion (35) and second proximal balloon portion (36), and wherein the second balloon element (32) has a first constriction (38) in the second central balloon portion (37), which reduces the balloon element circumference in the inflated state in comparison with the portions immediately to the right and left of the constriction (38),
wherein the first proximal balloon portion (26) of the first balloon element (22) and the second distal balloon portion (35) of the second balloon element (32) directly and flush with each other, or overlap or are configured in one piece.

2. Balloon catheter (10) according to claim 1, **characterized in that** the catheter body (11) has markings (40) in a region between the proximal end portion (16) and distal end portion (15) for indicating the position of the balloon catheter (10) in an insertion cannula.

3. Balloon catheter (10) according to claim 1 or 2, **characterized in that** in the proximal end portion (16) in the transition from the catheter body (11) to the connecting element (18) there is arranged an anti-kink element (42) which is coaxially arranged above the catheter body (11), and which stiffens the catheter body (11) in the proximal end portion (16) and is firmly connected to the connecting element (18).

4. Balloon catheter (10) according to one of the preceding claims, **characterized in that** at least the first (20) and the second (19) connecting port comprise vacuum valves.

5. Balloon catheter (10) according to one of the preceding claims, **characterized in that** it further comprises a stylet element (44) which is fixed in the catheter body (11) via the first (23) or second (33) lumen for stiffening the catheter body (11) therein.

6. Balloon catheter (10) according to one of the ceding claims, **characterized in that** the first balloon element (22) is fixed, preferably welded, in its distal balloon portion (25) by a partial inversion of the distal balloon portion (25) inwards and by a fixation of the inwards inverted distal balloon portion to the catheter body (11).

7. Balloon catheter (10) according to one of the preceding claims, **characterized in that** the proximal balloon portion (26) of the first balloon element (22) and the distal balloon portion (35) of the second balloon element (32) are fixed, preferably welded, in overlapping manner to the catheter body (11).

8. Balloon catheter (10) according to one of the preceding claims, **characterized in that** the first and/or second balloon element (22; 32) comprises or consists of a material selected from polyurethane, polyethylene, teflon, nylon.

9. Balloon catheter (10) according to one of the preceding claims, **characterized in that** the first lumen (23) has a substantially circular cross-section.

10. Balloon catheter (10) according to one of the preceding claims, **characterized in that** the second lumen (33) has a substantially oblong-hole-like cross-section.

11. Balloon catheter (10) according to one of the preceding claims, **characterized in that** the proximal balloon portion (26) of the first balloon element (22) and/or the proximal balloon portion (36) of the second balloon element (32) has a conical shape, the reduced end of the conical shape being arranged facing in the proximal direction (50).

12. Balloon catheter (10) according to one of the preceding claims, **characterized in that** it is dimensioned such that it can be advanced over and through an insertion cannula with its distal end portion (15) comprising the balloon elements (22; 32) into a vertebral body and is configured in the vertebral body for compressing spongy bone material.

13. Set for the treatment of vertebral body diseases or injuries, **characterized in that** it comprises the balloon catheter (10) according to one of the preceding claims and at least one of the following: a balloon adapter, an insertion cannula, a volume syringe, an access device.

## Revendications

1. Cathéter à ballonnet (10) pour la compression de matière osseuse spongieuse, le cathéter à ballonnet (10) comprenant les éléments suivants :
- un corps de cathéter flexible allongé (11) ayant une extrémité proximale (12) et distale (13), ainsi qu'un axe longitudinal (14) s'étendant entre l'extrémité proximale (12) et distale (13), et une zone d'extrémité distale (15) et proximale (16) ;
- un élément de raccordement (18), qui est relié de manière fixe avec la zone d'extrémité proximale (16) du corps de cathéter (11), et qui comprend au moins deux ou davantage de raccords (19, 20) ;
- un premier élément ballonnet gonflable et dégonflable (22), qui est gonflable à un premier diamètre en un état gonflé, et qui est agencé coaxialement autour du corps de cathéter (11) au niveau de son extrémité distale (13), le premier élément ballonnet (22) étant gonflable par l'intermédiaire d'une première lumière (23) par l'intermédiaire d'un fluide introduit dans celui-ci, la première lumière (23) s'étendant à partir de la zone d'extrémité distale (15) le long de l'axe longitudinal (14) du corps de cathéter (11) et jusqu'à l'extrémité proximale (12) dans un premier raccord (20), la première lumière (23) comprenant une ouverture (24) s'ouvrant dans l'espace intérieur du premier élément ballonnet (22) ; et, par ailleurs, le premier élément ballonnet (22) présentant une première zone de ballonnet distale (25), une première zone de ballonnet proximale (26) et une première zone de ballonnet intermédiaire (27) située entre celles-ci, le premier élément ballonnet (22) étant fixé au corps de cathéter (11) par l'intermédiaire de la première zone de ballonnet distale (25) et de la première zone de ballonnet proximale (26), et le premier élément ballonnet (22) présentant un premier rétrécissement (28) dans la première zone de ballonnet intermédiaire (27), qui réduit la circonférence de l'élément ballonnet à l'état gonflé en comparaison des zones de la zone de ballonnet intermédiaire (27) situées immédiatement à droite et à gauche du rétrécissement (28) ;
- un deuxième élément ballonnet gonflable et dégonflable (32), qui est gonflable à un deuxième diamètre dans un état gonflé, et qui est agencé coaxialement autour du corps de cathéter (11) au voisinage direct et à proximité du premier élément ballonnet (22), le deuxième élément ballonnet (32) étant gonflable par l'intermédiaire d'une deuxième lumière (33) par l'intermédiaire d'un fluide introduit dans celui-ci, la deuxième lumière (33) s'étendant à partir de la zone d'extrémité distale (15) le long de l'axe longitudinal (14) du corps de cathéter (11) et jusqu'à l'extrémité proximale (12) dans un deuxième raccord (19), la deuxième lumière (33) comprenant une ouverture (34) s'ouvrant dans l'espace intérieur du deuxième élément ballonnet (32) ; et, par ailleurs, le deuxième élément ballonnet (32) présentant une deuxième zone de ballonnet distale (35), une deuxième zone de ballonnet proximale (36) et une deuxième zone de ballonnet intermédiaire (37) située entre celles-ci, le deuxième élément ballonnet (32) étant fixé au corps de cathéter (11) par l'intermédiaire de la deuxième zone de ballonnet distale (35) et de la deuxième zone de ballonnet proximale (36), et le deuxième élément ballonnet (32) présentant un premier rétrécissement (38) dans la deuxième zone de ballonnet intermédiaire (37), qui réduit la circonférence de l'élément ballonnet à l'état gonflé en comparaison des zones situées immédiatement à droite et à gauche du rétrécissement (38) ;
la première zone de ballonnet proximale (26) du premier élément ballonnet (22) et la deuxième zone de ballonnet distale (35) du deuxième élément ballonnet (32) étant adjacentes l'une à l'autre directement et en affleurement, ou se chevauchant ou étant configurées d'un seul tenant.

2. Cathéter à ballonnet (10) selon la revendication 1, **caractérisé en ce que** le corps de cathéter (11) comprend des marquages (40) dans une zone entre la zone d'extrémité proximale (16) et distale (15), destinés à indiquer la position du cathéter de ballonnet (10) dans un tube guide.

3. Cathéter à ballonnet (10) selon la revendication 1 ou 2, **caractérisé en ce qu'**un élément anti-courbure (42) raidissant le corps de cathéter (11) dans la zone d'extrémité proximale (16), agencé coaxialement au-dessus du corps de cathéter (11), est agencé dans la zone d'extrémité proximale (16) dans la transition depuis le corps de cathéter (11) vers l'élément de raccordement (18), qui est relié de manière fixe avec l'élément de raccordement (18).

4. Cathéter à ballonnet (10) selon l'une quelconque des revendications précédentes, **caractérisé en ce qu'**au moins le premier (20) et le deuxième (19) raccord comprennent des clapets sous vide.

5. Cathéter à ballonnet (10) selon l'une quelconque des revendications précédentes, **caractérisé en ce qu'**il comprend en outre un élément stylet (44), qui est fixé dans le corps de cathéter (11) pour le raidissement de celui-ci par l'intermédiaire de la première (23) ou deuxième (33) lumière.

6. Cathéter à ballonnet (10) selon l'une quelconque des revendications précédentes, **caractérisé en ce que** le premier élément ballonnet (22) est fixé, de préférence soudé, au corps de cathéter (11) dans sa zone de ballonnet distale (25) par un retroussement partiel de la zone de ballonnet distale (25) vers l'intérieur et une fixation de la zone de ballonnet distale retroussée vers l'intérieur.

7. Cathéter à ballonnet (10) selon l'une quelconque des revendications précédentes, **caractérisé en ce que** la zone de ballonnet proximale (26) du premier élément ballonnet (22) et la zone de ballonnet distale (35) du deuxième élément ballonnet (32) sont fixées, de préférence soudées, au corps de cathéter (11) en chevauchement.

8. Cathéter à ballonnet (10) selon l'une quelconque des revendications précédentes, **caractérisé en ce que** le premier et/ou deuxième élément ballonnet (22 ; 32) comprend un matériau ou est constitué par celui-ci, qui est choisi parmi le polyuréthane, le polyéthylène, le téflon, le nylon.

9. Cathéter à ballonnet (10) selon l'une quelconque des revendications précédentes, **caractérisé en ce que** la première lumière (23) présente une section transversale essentiellement circulaire.

10. Cathéter à ballonnet (10) selon l'une quelconque des revendications précédentes, **caractérisé en ce que** la deuxième lumière (33) présente une section transversale essentiellement de type trou allongé.

11. Cathéter à ballonnet (10) selon l'une quelconque des revendications précédentes, **caractérisé en ce que** la zone de ballonnet proximale (26) du premier élément ballonnet (22) et/ou la zone de ballonnet proximale (36) du deuxième élément ballonnet (32) présentent un tracé en forme de cône, l'extrémité réduite du tracé en forme de cône étant agencée orientée dans la direction proximale (50).

12. Cathéter à ballonnet (10) selon l'une quelconque des revendications précédentes, **caractérisé en ce qu'**il est dimensionné de telle sorte qu'il puisse coulisser par l'intermédiaire d'un tube guide et au travers de celui-ci avec sa zone d'extrémité distale (15) comprenant les éléments ballonnets (22 ; 32) dans un corps vertébral et qu'il soit configuré pour comprimer une matière osseuse spongieuse dans le corps vertébral.

13. Ensemble pour le traitement de maladies ou blessures de corps vertébraux, **caractérisé en ce qu'**il comprend le cathéter à ballonnet (10) selon l'une quelconque des revendications précédentes, ainsi qu'au moins un des éléments suivants : un adaptateur de ballonnet, un tube guide, une seringue volumétrique, un dispositif d'accès.
